# EUROPEAN PATENT APPLICATION

(11) **EP 4 151 214 A1**
(43) Date of publication of application: **22.03.2023**
(21) Application number: 22196616.1
(22) Date of filing: 20.09.2022
(51) Int. Cl.: A61K 31/4709, A61P 31/14

(54) **RIMEGEPANT FOR TREATMENT OF HUMAN CORONAVIRUS INFECTION**

(30) Priority: 20.09.2021 PT 2021117473
(71) Applicant: UNIVERSIDADE NOVA DE LISBOA, 1099-085 Lisboa (PT)
(72) Inventor: TEIXEIRA SARAMAGO, ANA MARGARIDA, 1675-168 PONTINHA (PT); GONÇALVES MATOS LUÍS, RUTE MARGARIDA, 2775-623 CARCAVELOS (PT); PAIS DE FARIA DE ANDRADE ARRAIANO, CECÍLIA MARIA, 1990-122 LISBOA (PT); SILVA SOUZA, CAIO, 2780-109 OEIRAS (PT); PEREIRA LOUSA, DIANA ANDREIA, 2775-800 CARCAVELOS (PT); SIMÕES LOUREIRO NUNES SOARES, CLÁUDIO MANUEL, 2775-800 CARCAVELOS (PT); SOUSA PINTO TORRES FEVEREIRO, MIGUEL AGOSTINHO, 1200-856 LISBOA (PT); FERREIRA HENRIQUES DE OLIVEIRA MOURÃO, ANA MARGARIDA, 2560-286 TORRES VEDRAS (PT)
(74) Representative: Schulz Junghans Patentanwälte PartGmbB

(57) **Abstract**

The invention relates to the use of rimegepant or a pharmaceutically acceptable salt thereof for use in the treatment of infection of human coronavirus, namely SARS-CoV-2 and its variants (e.g. alpha, beta, gamma, delta, omicron, and others).

## Description

The present invention relates to the use of rimegepant for treatment of human coronavirus infection.

### Background of the Invention

The human coronavirus (hCoV) pandemic, caused by SARS-Coronavirus-2 (SARS-CoV-2), dramatically affects individuals and societies across the world. We currently lack a universally applied vaccine and effective anti-viral medication. Moreover, the longevity of the immunity provided by currently available vaccines as well as their efficiency in preventing infections with the novel strains of coronavirus remains unknown. The inhibition of early disease onset after onset of symptoms in individuals with high risk of a grave course of COVID-19 is key to protect lives and guard against overload of the health care systems. Currently, there is no effective clinical treatment against this disease. The only fully FDA-approved therapy for COVID-19 is Remdesivir, and so far, it is still the only one. However, the FDA authorized the emergency use of Paxlovid and a small number of monoclonal antibodies, which are expensive to produce and not all of them have real efficacy. To reduce COVID-19 mortality and disease burden on societies, anti-viral treatments and vaccination are urgently needed.

Furthermore, previous appearances of highly infectious coronaviruses in humans that are transmissible between human patients and lead to high mortality rates, such as SARS-CoV-1 and Middle East respiratory syndrome (MERS), show that it would be useful to have medication with a broad applicability to human coronavirus infection ready in the case of another unrelated emergence of a novel coronavirus that is infectious and transmissible in humans.

Rimegepant (BMS-927711, CAS Number: 1289023-67-1) is an oral Calcitonin gene-related peptide (CGRP) receptor antagonist. The compound was approved by FDA in February, 2020 for the acute treatment of migraine in adults. This is the first CGRP receptor antagonist available in a fast-acting orally disintegrating tablet (ODT) with effect lasting up to 48h after a single dose. Rimegepant offers an alternative treatment for those who experience inadequate efficacy, poor tolerability or have a contraindication to current acute therapies. Rimegepant is not an opioid and does not have addiction potential.

Based on the above-mentioned state of the art, the objective of the present invention is to provide means and methods to treat human coronavirus infections, including SARS-CoV-2, in human patients. This objective is attained by the subject-matter of the independent claims of the present specification, with further advantageous embodiments described in the dependent claims, examples, figures and general description of this specification.

### Summary of the Invention

The present invention relates to the drug rimegepant, 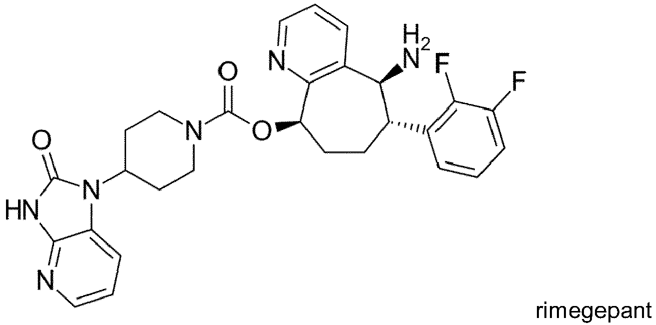 or a pharmaceutically acceptable salt thereof, particularly rimegepant sulfate, for use in the treatment of infection of human coronavirus, particularly infection by SARS-CoV-2 and its variants (e.g. alpha, beta, gamma, delta, omicron and others).

Rimegepantis the first drug that targets both coronavirus-specific RNA-degrading enzymes (RNases) at the same time, thus exerting a highly potent and specific antiviral action. At the same time, rimegepant will help trigger a robust immune response in the host to clear the virus.

Rimegepant has been found to inhibit the activity of two viral RNA nucleases: nonstructural protein 14 (nsp14) and nonstructural protein 15 (nsp15), both of which are crucial for the life cycle of coronaviruses. Nsp14 is an N-terminal 3'-5' exoribonuclease (ExoN) responsible for viral RNA proofreading avoiding lethal mutagenesis, and a C-terminal N7-methyltransferase (N7-MTase) involved in attaching a 5'-cap to the viral RNA, so that the viral RNA is able to use the host translation machinery and is not recognized as foreign. Nsp15 is a uridine-specific endoribonuclease responsible for destroying U-rich intermediate RNAs that function as activators of the host immune system. Both proteins are essential for virus amplification, are found exclusively in the genome of coronaviruses, and function as major interferon antagonists in SARS-CoV-2 (Yuen et al CK Emerg Microbes Infect. 2020, 9:1418-1428).

Rimegepant has been identified as a potential inhibitor of nsp14 and nsp15 in a docking screen of a library of 8368 drugs, either experimental or already approved for other conditions. These target proteins are highly conserved among coronaviruses. Thus, compounds found to have the ability of inhibiting SARS-CoV-2 RNases are expected to be active across the family *Coronaviridae,* extending their therapeutic potential to other clinically relevant coronaviruses (Yoshimoto et al 2020. Protein J. 39(3): 198-216; Saramago, M., et al. The FEBS Journal 2021, 288: 5130-5147; Saramago M., et al. Microorganisms 2022, 210(2):342022).

The enormous mutating ability of SARS-CoV-2 makes it more potent than previous coronavirus strains. Such variation capacity originates new strains with different infection patterns. Different mutant strains of this virus are constantly being identified in distinct parts of the world. The different variants of SARS-CoV-2 that have emerged are highly associated with multiple mutations in structural proteins, especially in the Spike protein. For these reasons, the present invention consists of a therapy against highly conserved targets (nsp14 and nsp15).

Many individuals (including young people) lose the battle to COVID-19, which is known to cause a systemic inflammatory response. Excessive production of pro-inflammatory cytokines is an event known as "cytokine storm" correlated directly with lung injury, septic shock with multi-organ failure, and unfavorable prognosis of severe COVID-19. The interleukin-6 (IL-6) is the most frequently reported cytokine to be increased in COVID-19 patients. It has been associated to higher mortalities and it is a marker for severity assessment (Ragab et al 2020 Front. Immunol.11: 1446).

Calcitonin gene-related peptide (CGRP) is known to enhance cytokine-dependent II-6 production (Sakuta et al 1995 Cell Immunol 165:20-25). It was hypothesized that the excess release of the neuropeptide CGRP might contribute to abnormal vascular reactivity observed in acute lung injury (Lange et al 2009 J Appl Physiol 107: 176-184). Therefore, treatments that block the CGRP pathway (e.g. Rimegepant) might be beneficial and help mitigate the hyperinflammatory response in severe cases of COVID-19.

In addition to the aforementioned, the present invention demonstrates that rimegepant has the ability to efficiently suppress the viral propagation of SARS-CoV-2 in cell culture. This means that this CGRP receptor antagonist also exerts an antiviral action against Coronavirus, through the inhibition of the RNases nsp15 and nsp14.

### Terms and definitions

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising," "having," "containing," and "including," and other similar forms, and grammatical equivalents thereof, as used herein, are intended to be equivalent in meaning and to be open ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictate otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a," "or," and "the" include plural referents unless the context clearly dictates otherwise.

### Detailed Description of the Invention

Administration of rimegepant is a clinically applicable treatment against human coronaviruses and their disease, including COVID-19 with post-exposure efficacy.

The inventors propose to use rimegepant in two post exposure modalities, a low dosage modality of 75 mg, twice daily against virus propagation, and a regular dosage of 75 mg more than twice daily (up to 600 mg for 14 days) in more severe / persistent cases for enhanced inhibition of viral proteins.

The inventors propose to use liquid compositions in a unit dosage range of 1-100 mg/mL, when administered intravenously.

The inventors propose that targeting the virus-specific RNA nucleases confers significant advantages in hindering the amplification of the virus while posing little risks to the host. This is distinct from anti-viral agents, such as Remdesivir - the only FDA approved treatment against COVID-19. Remdesivir is an antiviral drug that belongs to the type of nucleoside inhibitors (NAs) that stalls the coronaviruses' RNA-dependent RNA polymerase. This drug has a conserved mode of inhibition across different viruses, where a single point mutation in the Ebolavirus polymerase leads to viral drug resistance. The use of NAs in Coronaviruses is challenging due to the presence of the nsp14 ExoN proofreading activity. Nsp14 significantly attenuates the inhibitory effect of NA drugs that function through premature termination of viral genome replication. Indeed, WHO has issued a conditional recommendation against the use of remdesivir in hospitalized patients, regardless of disease severity, as there is no meaningful effect on mortality or on other important outcomes for patients, such as the need for mechanical ventilation or time to clinical improvement.

The inventors further propose that rimegepant is used in patients showing initial to severe symptoms of COVID-19.

The inventors also propose that rimegepant, by lowering the viral load, may decrease the patient's chances of developing the persistent symptoms associated with long-COVID (fatigue, brain fog, headache, shortness of breath, joint and chest pain, etc.).

Rimegepant is currently used for the acute treatment of migraine in adults, by blocking CGRP receptors. CGRP is a neuropeptide involved in migraine pathophysiology. It is also known to play a role in the respiratory system and in innate immune activation during infection (Camp et al 2021 Int J Mol Sci 22: 5699). This neuropeptide was shown to be widely distributed in the nervous system, cardiovascular system and lungs (Hay et al 2017 Headache. 57(4):625-36).

Viral infections activate CGRP release, contributing to acute and chronic lung injury. Therefore, antagonizing CGRP is a very potent therapeutic concept. A CGRP receptor antagonist may help mitigate the severe inflammatory response at the alveolar level, blocking the cascade that leads to oxygen desaturation and acute respiratory distress syndrome.

As already mentioned, CGRP is known to enhance cytokine-dependent II-6 production (Sakuta et al 1995 Cell Immunol. 165:20-25). Stronger inflammatory response is also associated with headache in hospitalized COVID-19 patients with moderate disease severity. Increased levels of IL-6 may play a role in the induction of the trigeminal system and manifestation of headache associated to SARS-CoV-2 infection (Bolay et al 2021. The Journal of Headache and Pain 22: 94). Accordingly, serum levels of IL-6 were detected to be significantly higher in COVID-19 patients with severe headache, compared to COVID-19 patients without headache (Bolay et al 2021. The Journal of Headache and Pain 22: 94). Additionally, COVID-19 patients with severe headache showed a higher ratio of pulmonary involvement diagnosed by thorax Computed tomography (CT) imaging compared to COVID-19 patients without headache (65% vs. 33 %, p<0.001) (Bolay et al 2021. The Journal of Headache and Pain 22: 94). Since rimegepant is a CGRP receptor antagonist, it may help mitigate the headache and also the hyperinflammatory response associated to COVID-19, reducing the need for supplemental oxygenation and artificial ventilation.

According to the American Migraine Foundation, with the alpha, delta and omicron variants, a migraine-like headache has been a common symptom of SARS-CoV-2 infection. This headache may persist after infection being a sign of post-COVID. Many patients with a history of migraine report having a very similar headache that does not respond to their usual medications. Others report that their COVID-related headaches are different, more severe, or longer lasting. The treatment often includes non-steroidal anti-inflammatory drugs, triptans, opioids and combination medications. The advantage of rimegepant over these pharmacologic agents is that it can be used for the treatment of the attack and for prevention.

A multicentre cross-sectional study, published by the Spanish Society of Neurology, reported that using anti-CGRP monoclonal antibodies does not increase the risk of COVID-19, or worsen prognosis in those who get the infection (Caronna et al 2021 Neurologia).

Inhibiting endogenous CGRP signaling has been shown to attenuate respiratory malfunction in ovine models of burn and smoke inhalation and mice models of acid-induced lung injury (Lange. 2009. 107:176-184; Aoki-Nagase. 2007. 12:807-813).

The *in vitro* experiments presented herein led the inventors to conclude that the optimal concentration for rimegepant is 175 µM (93 µg/ml). This optimal drug concentration was defined as the concentration of inhibitor that had the lowest toxic effect and simultaneously most protected cells from infection by SARS-CoV-2. 175 µM of rimegepant had an antiviral effect for 72h.

Rimegepant is an FDA-approved and its recommended dose for the treatment of migraines is 75 mg over a 24h period. However, Tong *et al* reported that rimegepant have been well tolerated in healthy subjects who received single doses of rimegepant up to 1500 mg, compared to individuals that received placebo. Also, multiple doses of rimegepant up to 600 mg for 14 days were well tolerated without significant adverse events (Tong et al 2013 The Journal of Headache and Pain2013,14(Suppl 1):P118).

Rimegepant is the first and only CGRP receptor antagonist available in the form of orally disintegrating tablets and the only oral CGRP receptor antagonist with effect lasting up to 48h after a single dose.

Following oral administration of the orally disintegrating tablet, maximum plasma concentrations were achieved at 1.5 hours (Tₘₐₓ) (https://www.accessdata.fda.gov/drugsatfda docs/label/2021/212728s006lbl.pdf).

During Phase I clinical trials, a maximum tolerated dose (MTD) was not found, despite achieving plasma concentrations that were 50 times higher than those observed near the low predicted therapeutic dose of 75 mg (maximum serum concentrations - Cₘₐₓ 784 ng/mL and concentration of drug circulating in plasma, blood or serum - AUC 3.729 ng*h/mL). Therefore, studies defined the safety of 25 to 1,500 mg as well tolerated.

Approximately 77% of an administered dose is eliminated unchanged with no major metabolites (i.e., > 10%) detected in plasma (https://www.accessdata.fda.gov/drugsatfda docs/label/2021/212728s006lbl.pdf).

The elimination half-life of rimegepant is approximately 11 h, and it is cleared mainly by the fecal and renal route (https://www.accessdata.fda.gov/drugsatfda docs/label/2021/212728s006lbl.pdf).

Rimegepant does not require dose adjustment in patients with mild, moderate, or severe renal impairment nor does it require dose adjustment in patients with mild or moderate hepatic impairment (https://www.accessdata.fda.gov/drugsatfda docs/label/2021/212728s006lbl.pdf).

Rimegepant has a good long-term safety profile, being generally well tolerated, with no suggestion of systemic toxicities, with a bioavailability of 64% (https://go.drugbank.com/drugs/DB12457).

The present invention is a starting point to repurpose rimegepant, a currently FDA-approved drug for the treatment of migraines in adults, which appears to be safe and to have advantages over the COVID-19 therapeutic available so far.

Rimegepant's potential should not be limited to headache/migraine symptoms. CGRP receptor antagonists, such as rimegepant, may also be useful pharmacological agents for other CGRP disorders, such as asthma, and other inflammatory conditions, typical of COVID-19. Indeed, this drug may have benefits to COVID-19 symptoms in general, as the inventors demonstrate that rimegepant blocks the activity of SARS-CoV-2 RNases nsp14 and nsp15, two crucial and highly conserved targets in SARS-CoV-2 and its variants. Consequently, the drug has also the ability to impair viral replication in Vero E6 cells.

Rimegepant may thus exert a triple effect by inhibiting viral replication, by being a CGRP receptor antagonist, and by treatment of migraines associated to COVID-19.

### General treatment criteria

The SARS-CoV-2 positive patient to be treated with rimegepant presents at the hospital with the most common COVID-19 symptoms, including
- Respiratory illness symptoms (sore throat, dry cough, shortness of breath, chest pain)
- High temperature
- Sudden loss of sense of smell and/or taste

Possibly other symptoms are also present, such as
- Headache
- General weakness, unwellness
- Aching muscles
- Gastrointestinal symptoms (nausea, vomiting, diarrhea, stomachache)
- Head cold
- Skin rash
- Sore eyes

### High dosage (more than twice daily)

Patients to whom one or more of the risk criteria apply will receive the high dose treatment:
- age >60 years;
- high blood pressure;
- cardiovascular disease;
- diabetes (particularly diabetes mellitus type II);
- chronic respiratory diseases;
- obesity class III (morbid, BMI greater than or equal to 40 kg/m2).

### TREATMENT DURATION

Treatment should last for 10-20 days with intermittent presentation at the hospital for blood tests, including immune cell analyses, cytokines and oxygenation levels, as well as viral titer determination.

The data provided in here demonstrate that rimegepant exerts an antiviral effect on SARS-CoV-2. Rimegepant inhibits SARS-CoV-2 propagation by non-competitive inhibition of RNA nucleases nsp14 (a 3'-5' exoribonuclease and guanine-N7-methyl-transferase acting in coronavirus replication and transcription) and nsp15 (an endoribonuclease that preferentially cleaves 3' of U residues avoiding immune system activation) (Saramago, M., et al. The FEBS Journal 2021, 288: 5130-5147; Saramago M., et al. Microorganisms 2022, 210(2):342022). Indeed, both enzymes are essential and were identified as major interferon antagonists from SARS-CoV-2 (Yuen et al CK Emerg Microbes Infect. 2020 9:1418-1428). Moreover, the FDA-approved rimegepant as a CGRP receptor antagonist may have beneficial and synergistic effects on SARS-CoV-2 replication / dissemination, and in COVID-19 inflammatory pathogenesis / disease progression.

### Medical treatment, Dosage Forms and Salts

The skilled person is aware that rimegepant may be present as a pharmaceutically acceptable salt of said drug. Pharmaceutically acceptable salts comprise the ionized drug and an oppositely charged counterion. Non-limiting examples of pharmaceutically acceptable cationic salt forms include aluminium, benzathine, calcium, ethylene diamine, lysine, magnesium, meglumine, potassium, procaine, sodium, tromethamine and zinc.

### Pharmaceutical Compositions and Administration

Another aspect of the invention relates to a pharmaceutical composition comprising rimegepant, and a pharmaceutically acceptable carrier.

In certain embodiments of the invention, the compound of the present invention is typically formulated into pharmaceutical dosage forms to provide an easily controllable dosage of the drug and to give the patient an elegant and easily handleable product.

Typical routes of administering the pharmaceutical compositions of rimegepant include, without limitation, oral, topical, transdermal, inhalation, sublingual, buccal, rectal, vaginal, intranasal, subcutaneous injections, intravenous, intramuscular, intrasternal injection or infusion technique.

### Method of Manufacture and Method of Treatment according to the invention

The invention further encompasses, as an additional aspect, the use of rimegepant as identified herein for use in a method of manufacture of a medicament for the treatment or prevention of human coronavirus infection, namely COVID-19.

Similarly, the invention encompasses methods of treatment of a patient having been diagnosed with human coronavirus infection, namely COVID-19. This method entails administering to the patient an effective amount of rimegepant.

The invention is further illustrated by the following examples and figures, from which further embodiments and advantages can be drawn. These examples are meant to illustrate the invention but not to limit its scope.

### Description of the Figures

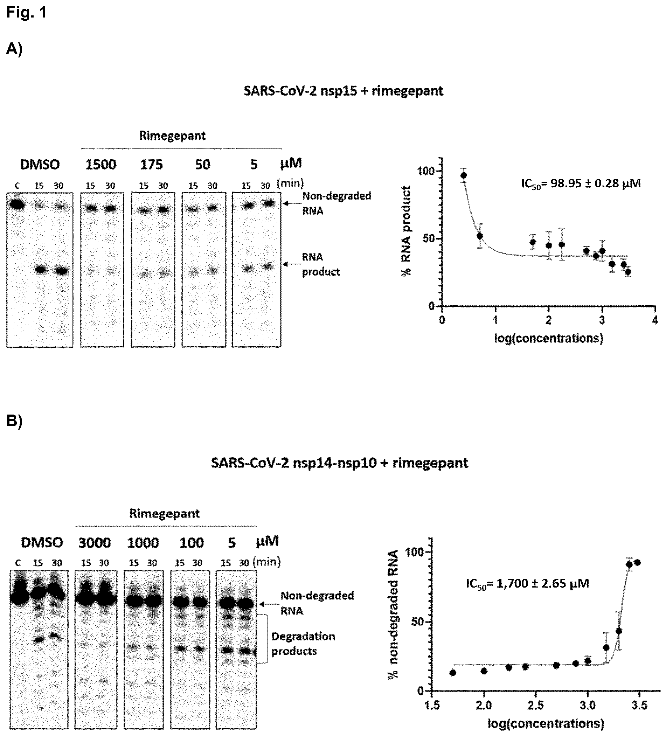

- Fig. 1: shows the degradation of RNA based on activity of both nsp15 and nsp14 in response to varying concentrations of rimegepant.
A synthetic 22-mer oligoribonucleotide (H4, see Saramago et al. FEBS J. 288(17) 2021, 5130-5147) was used as a substrate for nsp14 and a 16-mer oligoribonucleotide (Ortiz-Alcantara et al., Virus Adaption and Treatment, 2010; Sareamago, Microorganisms 2022, ibid.) for nsp15. Both RNAs were labelled at their 5' end with [³²P]-γ-ATP and T4 Polynucleotide Kinase (Ambion) in a standard reaction. MicroSpin G-50 columns (Cytiva) were used to remove excess [³²P]-γ-ATP. The synthetic RNAs were resuspended in 10 mM of Tris-HCI pH 8.0 and incubated 10 min at 80°C followed by 45 min at 37°C.
Different concentrations (µM) of rimegepant were incubated with 300 nM of nsp15 or with 40 nM-160 nM of nsp14-nsp10 proteins (7 minutes at room temperature), before initiating the *in vitro* assay through the addition of the P³²-RNA substrate (50 nM). The activity assays were performed in a final volume of 12 µl containing the activity buffer 50 mM HEPES pH 7.4, 1 mM DTT, 5 mM MgCl₂ in case of nsp14-nsp10 complex, and 25 mM HEPES pH 7.4, 50 mM NaCl, 1 mM DTT, 10 mM NaHPO₄, 5 mM MnCl₂ for nsp15. The reactions were incubated at 37°C, and then aliquots of 4 µl were withdrawn at the time-points indicated in the respective images. The reactions were stopped by adding a gel-loading buffer containing 80% (v/v) formamide and 10 mM EDTA. Rimegepant was solubilized and diluted in DMSO, whose concentration was kept constant and corresponds to the maximum amount of rimegepant tested in the assay. Reaction controls were performed with the RNAs (without the enzymes), with either activity buffer or buffer supplemented with DMSO. Controls were incubated in the same conditions during the assay. Reaction products were resolved in a 20% denaturant polyacrylamide gel (7M urea). Signals were visualized by PhosphorImaging (TLA-5100 Series, Fuji). All the experiments were performed at least in triplicate.
Inhibition profile curves were calculated by quantification of gel-based RNase assays. Data were plotted against log₁₀ of [rimegepant] and dose response curves were generated using non-linear regression (GraphPad Prism 8 software). At least three biological replicates were used for each concentration.

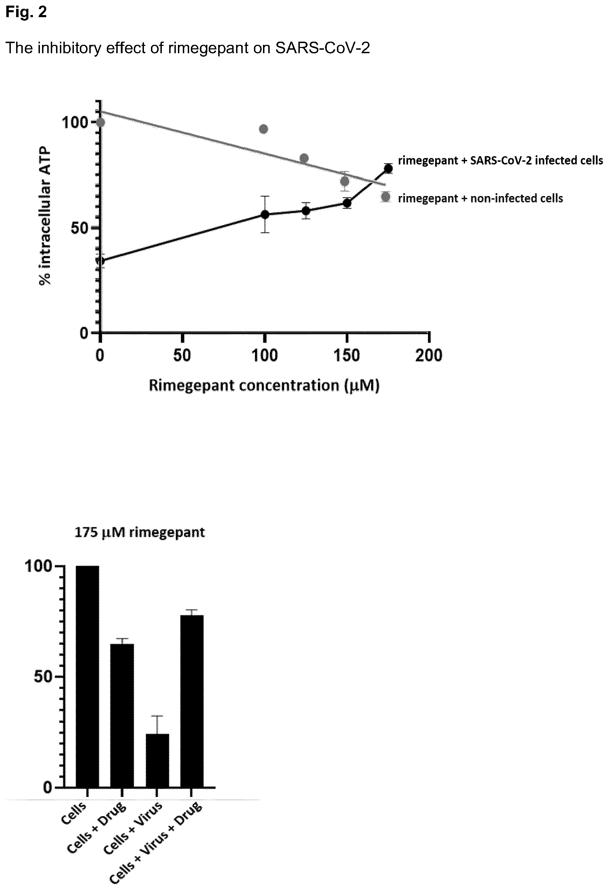

- Fig. 2: shows bioluminescence of cells either control or infected with SARS-CoV-2, and untreated or treated with rimegepant. The bioluminescence signal corresponds directly to the amounts of intracellular ATP and reflects viability of cells. The inhibitory effect of rimegepant on SARS-CoV-2 multiplication in Vero E6 cells was evaluated. Briefly, Vero E6 cells were harvested and suspended in MEM medium supplemented with 10% fetal bovine serum (FBS), penicillin (100 U/ml) and streptomycin (100 mg/ml). Different concentrations of rimegepant were mixed with 3 × 10⁴ Vero E6 cells and platted into 96-well cell culture plates (100 µl/well). SARS-CoV-2 (30 PFUs/25 µl) was then added to Vero-E6 cells and the plates were incubated at 37°C in a 5% CO₂ atmosphere for 72h. After that period plates were examined for the presence or absence of cytopathic effect (CPE) under a light microscope. In addition, the viability of cells in culture was assessed by measuring intracellular ATP levels, using a luminescent assay as described above (CellTiter-Glo^{®} Luminescent Cell Viability Assay). The luminescent signal is proportional to the amount of ATP present and the optimal drug concentration was chosen as the concentration of drug that most protected cells from infection by SARS-CoV-2, having simultaneously lower effect in cell viability in the absence of virus. Cellular ATP levels in the presence of various concentrations of rimegepant and the ability of the drug to recover cells from SARS-CoV-2 infection were plotted using GraphPad Prism 8 software.

### Examples

### Example 1

AutoDock Vina (Trott and Olson, Journal of Computational Chemistry, 2010 31(2), 455-461) was used to analyze the interaction of several sets of compounds with SARS-CoV-2 nsp14 and nsp15. Drugs were retrieved from Drugbank (doi: 10.1093/nar/gkx1037) datasets: FDA approved, Experimental, Investigational and Nutraceutical. Ligand files were prepared for docking using AutoDockTools 1.5.6 utilities by assigning gasteiger charges, AD4 atom types and rotable bonds. The protein structures found in PDB IDs 6VWW (doi: 10.2210/pdb6VWW/pdb and doi: 10.1002/pro.3873) and 6WXC (doi: 10.2210/pdb6WXC/pdb and doi: 10.1038/s42003-021-01735-9) were used for the nsp15 screenings. All non-proteic compounds were removed except for the phosphate ion that was present in the active site of 6WXC. Since the structure of SARS-CoV-2 nsp14 RNase was not available at the time the inventors performed the dockings, and given the very high identity with the homologous protein of SARS-CoV-1, they used a homology-model previously built in-house (Saramago, M. ibid.). However, the structure already available corroborates their model (Moeller, NH et al PNAS 2022, 119(9): e2106379119). All proteins were prepared for docking calculations using AutoDockTools by computing gasteiger charges and assigning AD4 atom types. No flexibility was taken into account in the receptor structures.

Plasmids expressing nsp10, nsp14 and nsp15 were obtained as described by Saramago, M ibid. Nsp10, nsp14 and nsp5 were expressed and purified as previously described (Saramago M., et al. Microorganisms 2022, 210(2):342022).

Synthetic RNAs already described in the literature were used and labeled at their 5' end with [γ-³²P]-ATP (Bouvet et al PNAS 2012: 109: 9372-9377; Ortiz-Alcantara et al Virus Adaptation and Treatment 2010:2 125-133; Saramago, M. ibid.). In the assays with nsp14-nsp10 protein complex, the H4 RNA was used. It consists in a 22-nucleotide (nt) long RNA with a 5' single-stranded (ss) tail and a 3'-end engaged in a stable duplex structure (Saramago, M. ibid.). The nsp14 ExoN activity was then evaluated by analyzing the hydrolysis of the H4 RNA 5'-end radiolabeled with a ratio nsp14-nsp10 of 1:4 (Saramago, M. ibid.). The reaction products from this reaction range between 17- to 20-nts in length, which corresponds to cleavage from the 3'-end of the RNA duplex region (Saramago, M. ibid.). To test the activity of nsp15, a synthetic RNA previously reported in Ortiz-Alcantara et al. 2010 was used. This substrate is a 16-nt long RNA with a 5'-end with two ss-nucleotides followed by a stem-lop structure and a 3'-ss tail (Ortiz-Alcantara et al Virus Adaptation and Treatment 2010, 2 125-133). This RNA contains a unique U residue where the enzyme cleaves, generating two breakdown products. Since the RNA molecule is labelled at its 5'end, only the upstream degradation product is visible in the gel.

In the reactions performed, the RNA substrate and enzymes concentrations were kept constant. Reaction controls were performed in the absence of the enzymes, with buffer supplemented with DMSO, since this solvent was used to solubilize the drug. Rimegepant has an inhibitory effect on the activity of both viral RNases. To evaluate this effect over SARS-CoV-2 nsp15, a range of concentrations between 3,500 and 2.5 µM was tested (only some of the concentrations are represented in the figure). The inventors demonstrate that rimegepant is able to inhibit SARS-CoV-2 nsp15 activity with a IC₅₀ of 98.95 ± 0.28 µM. Regarding the nsp14-nsp10 protein complex, a range of concentrations between 3,500 and 50 µM was tested. Rimegepant is also able to block the exoribonucleolytic activity of nsp14 with an IC₅₀ of 1,700 ± 2.65 µM.

The results are shown in Figure 1.

### Example 2

Virus isolation was performed in biosafety level 3 (BSL3) facilities at INIAV- Laboratory of Virology. SARS-CoV-2 isolate (ICV1006) was obtained from a Portuguese COVID19 patient. The production of virus stock was accomplished in Vero E6 cells (African green monkey kidney cells, catalog no.ATCC CRL-1586) maintained in Eagle's minimum essential medium (MEM) supplemented with 10 % fetal bovine serum (FBS) and penicillin (100 U/ml) and streptomycin (100 mg/ml), at 37°C in a 5% carbon dioxide atmosphere. After three times freeze-thaw cycles the cultures were clarified at 1500 × g for 15 min at 5°C. The clarified supernatant was filtered through a 0.22 µm filter, aliquoted and kept at -80° C until used. The infectivity titer of the viral stock was determined by a standard plaque assay.

The potential cellular toxicity of rimegepant in Vero E6 cells was determined by measuring cellular ATP levels in the presence of various concentrations of the drug (100, 125, 150, 175, 200 and 250 µM) (Figure 2). To perform this, cytotoxicity in cell wells was measured 72 h after treatment with rimegepant, using the CellTiter-Glo luminescent cell viability assay kit (Promega) according to the manufacturer's instructions. Briefly, a total of 50 µl of medium were removed and discarded from each well and 50 µl of reconstituted CellTiter-Glo reagent were added. After 2 min agitation to promote cell lysis, the mixtures were transferred to an opaque plate and incubated for 10 minutes to stabilize the luminescent signal that was then measured in a Luminoscan Ascent Luminometer (Labsystems). The maximum nontoxic concentration was defined as the concentration of inhibitor that had minor or no effect on cellular ATP levels.

Rimegepant was also evaluated in cell infection assays with SARS-CoV-2. To accomplish this goal, Vero E6 cells were incubated with increasing concentrations of rimegepant in the presence of 30 pfu SARS-CoV-2. The virus was isolated from a Portuguese COVID-19 positive patient sample provided by the reference National Institute of Health (INSA, Portugal). The effect of rimegepant on SARS-CoV-2 replication was investigated in plaque formation assays and by measuring intracellular ATP levels in viable cells using CellTiter-Glo assay (Promega).

As a control, ATP levels were measured on Vero E6 cells, and that value was considered 100% (Figure 3). ATP was also measured on cells infected with SARS-CoV-2. Obviously, there was a drastic decrease on the levels of ATP upon infection, indicating that there are less metabolically active cells. As already mentioned, in order to determine the toxicity of the drug, Vero E6 cells were incubated with different concentrations of rimegepant (100, 125, 150, 175, 200 and 250 µM). The optimal drug concentration was defined as the concentration of rimegepant that had the lowest or no effect on cellular ATP levels in the absence of virus, and simultaneously most protected cells from infection by SARS-CoV-2. These experiments led to conclude that the optimal concentration for rimegepant is 175 µM (93 µg/ml). When 175 µM of this drug were added to Vero E6 cells infected with SARS-CoV-2, it was able to totally rescue the cells from the infection.

### 175 µM is the optimal concentration (lowest toxic effect / most protected the cells from the infection)

The results are shown in Figure 2.

### Example 3

Sequence alignment of nsp15 from MERS-CoV, SARS-CoV-1, SARS-CoV-2 Omicron BA.4, SARS-CoV-2, SARS-CoV-2 Alpha, SARS-CoV-2 Beta, SARS-CoV-2 Gamma, SARS-CoV-2 Omicron BA.1, SARS-CoV-2 Delta. Important residues for nsp15 RNase activity, specificity to degrade U residues and protein hexamerization, include N164, H235, H250, K290, S294, T341 and Y343 - in SARS-CoV-2 (Saramago M., et al. Microorganisms 2022, 210(2):342022). These residues are fully conserved in SARS-CoV-2 and its variants of concern, SARS-CoV-1 and MERS. In fact, nsp15 is fully conserved among the emerging variants of concern with no mutations recorded. Additionally, SARS-CoV-2 nsp15 shares 88.7% sequence identity and 94.8% of similarity with the SARS-CoV-1 homolog, and 50.1% sequence identity, and 63.9% similarity with the homolog from MERS-CoV. The same important residues maintain their conservation in SARS-CoV-1 and MERS-CoV.

A sequence alignment of nsp14 from MERS-CoV, SARS-CoV-1, SARS-CoV-2 Omicron BA.4, SARS-CoV-2, SARS-CoV-2 Alpha, SARS-CoV-2 Beta, SARS-CoV-2 Gamma, SARS-CoV-2 Omicron BA.1, and SARS-CoV-2 Delta can be performed. Nsp14 belongs to the superfamily of DEDD exonucleases, and these active site residues are recited in the preceding paragraph. These amino acids are distributed over three canonical motifs: motif I (D90/E92), motif II (D243) and motif III (D273) - numbering according to SARS-CoV-2. These important catalytic residues are fully conserved in SARS-CoV-2 and its variants of concern, SARS-CoV-1 and MERS-CoV (Saramago, M., et al. The FEBS Journal 2021, 288: 5130-5147). Indeed, nsp14 is fully conserved among the emerging variants of concern with only one mutation detected in the Omicron variants, the I42V. However, this amino acid is not located at the active center or at the interaction surface with the nsp10 protein with which nsp14 interacts to form a heterodimer. So, this mutation has no biological meaning. This information is supported by protein modelling data on the omicron nsp14 (data not shown). Additionally, SARS-CoV-2 nsp14 has high similarity with nsp14 from SARS-CoV-1 (95% of sequence identity and 99.1% of sequence similarity). Less conservation with the MERS-CoV amino acid sequence is found, but the crucial residues for the RNase activity of nsp14 maintain their conservation in SARS-CoV-1 and MERS-CoV.

This information confirms that rimegepant is equally effective in inhibiting the activity of the RNases nsp15 and nsp14 of the SARS-CoV-2 variants and thus exerting an antiviral effect. And most likely, it will also be effective against other human coronaviruses, namely SARS-CoV-1 and MERS-CoV.

## Claims

1. Rimegepant, or a pharmaceutically acceptable salt thereof, particularly rimegepant sulfate, for use in the treatment of infection of human coronavirus.

2. Rimegepant or a pharmaceutically acceptable salt thereof for use according to claim 1, wherein the infection is an infection by SARS-CoV-2.

3. Rimegepant or a pharmaceutically acceptable salt thereof for use according to claim 1, wherein the infection is an infection by an emerging variant of SARS-CoV-2, particularly a variant selected from the group consisting of SARS-CoV-2 alpha, SARS-CoV-2 beta, SARS-CoV-2 gamma, SARS-CoV-2 delta, SARS-CoV-2 omicron.

4. Rimegepant or a pharmaceutically acceptable salt thereof for use according to claim 1, 2 or 3, wherein the drug is administered at a dose that leads to a plasma level of 1-5 nmol/L.

5. Rimegepant or a pharmaceutically acceptable salt thereof for use according to claim 1, 2 or 3, wherein the drug is administered at a dose that leads to a plasma level of 5 nmol/L to 1 µmol/L.

6. Rimegepant or a pharmaceutically acceptable salt thereof for use according to claims 3, 4 or 5, wherein the drug is administered once daily / twice daily by oral administration form.

7. Rimegepant or a pharmaceutically acceptable salt thereof for use according to claims 4 or 5, wherein the drug is administered by systemic administration.

8. Rimegepant or a pharmaceutically acceptable salt thereof for use according to any one of the preceding claims, wherein rimegepant is formulated for administration as a tablet, a capsule, a nasal spray, a powder, as granules, as a solution, a suppository, by injection, as an inhalant, as a gel, as microspheres, or as an aerosol.

9. Rimegepant or a pharmaceutically acceptable salt thereof for use according to any one of the preceding claims 2 to 8, wherein the drug is administered to a patient showing initial symptoms of COVID-19 or having been diagnosed with COVID-19 in the 28 hours preceding the administration.

10. Rimegepant or a pharmaceutically acceptable salt thereof for use according to any of the preceding claims 2 to 9, wherein the drug is administered to a patient showing mild symptoms of COVID-19, particularly wherein the patient is normoxic, more particularly wherein the blood oxygenation level is equal or larger than 90%.

11. Rimegepant or a pharmaceutically acceptable salt thereof for use according to any of the 9, wherein the drug is administered to a patient showing moderate to severe symptoms of COVID-19, particularly wherein the patient is **characterized by** one or more of the following risk factors:
- age >60 years;
- high blood pressure;
- cardiovascular disease;
- diabetes (particularly diabetes mellitus type II);
- chronic respiratory diseases;
- obesity class III (morbid, BMI greater than or equal to 40 kg/m2)
and /or
the patient's blood oxygenation level is below 90%.

12. Rimegepant a pharmaceutically acceptable salt thereof for use according to any of the previous claims, wherein the drug is also used to treat to treat headache or migraines associated with COVID-19.
